# EUROPEAN PATENT SPECIFICATION

(11) **EP 1 571 202 B1**
(45) Date of publication and mention of the grant of the patent: **01.07.2009**
(21) Application number: 05004314.0
(22) Date of filing: 28.02.2005
(51) Int. Cl.: C12N 1/20, C12R 1/01, C02F 3/34

(54) **PVA-decomposing bacteria and method for decomposing PVA**
PVA-zersetzendes Bakterium und Methode zur Zersetzung von PVA
Bactérie décomposant l'alcool de polyvinylique (PVA) et procédé de décomposition PVA

(30) Priority: 01.03.2004 JP 2004055688
(43) Date of publication of application: 07.09.2005
(73) Proprietor: FUJIFILM Corporation, Minato-ku Tokyo (JP)
(72) Inventor: Kojima, Masayoshi, Minami-ashigara-shi Kanagawa 250-0193 (JP); Ikeda, Tsukasa, Utsunomiya-shi Tochigi 321-0904 (JP); Morohoshi, Tomohiro, Utsunomiya-shi Tochigi 321-0912 (JP)
(74) Representative: HOFFMANN EITLE

(56) References cited:
- DATABASE WPI Section Ch, Week 200436 Derwent Publications Ltd., London, GB; Class A35, AN 2004-379495 XP002331743 & JP 2003 250527 A (KAO CORP) 9 September 2003 (2003-09-09)
- KIM B C ET AL: "Degradation of polyvinyl alcohol by Sphingomonas sp. SA3 and its symbiote" JOURNAL OF INDUSTRIAL MICROBIOLOGY AND BIOTECHNOLOGY 01 JAN 2003 UNITED KINGDOM, vol. 30, no. 1, 1 January 2003 (2003-01-01), pages 70-74, XP002331737 ISSN: 1367-5435
- KAWAI F: "Sphingomonads involved in the biodegradation of xenobiotic polymers" JOURNAL OF INDUSTRIAL MICROBIOLOGY AND BIOTECHNOLOGY, vol. 23, no. 4-5, October 1999 (1999-10), pages 400-407, XP002331738 ISSN: 1367-5435
- TOKIWA YUTAKA ET AL: "A modified method for isolating poly(vinyl alcohol)-degrading bacteria and study of their degradation patterns" BIOTECHNOLOGY LETTERS, vol. 23, no. 23, December 2001 (2001-12), pages 1937-1941, XP002331739 ISSN: 0141-5492
- DAUGULIS ANDREW J ET AL: "Microbial degradation of high and low molecular weight polyaromatic hydrocarbons in a two-phase partitioning bioreactor by two strains of Sphingomonas sp." BIOTECHNOLOGY LETTERS, vol. 25, no. 17, September 2003 (2003-09), pages 1441-1444, XP002331740 ISSN: 0141-5492
- JANIKOWSKI T B ET AL: "Use of a two-phase partitioning bioreactor for degrading polycyclic aromatic hydrocarbons by a Sphingomonas sp" APPLIED MICROBIOLOGY AND BIOTECHNOLOGY, vol. 59, no. 2-3, July 2002 (2002-07), pages 368-376, XP002331741 ISSN: 0175-7598
- DATABASE EMBL SEQUENCE DATABASE EBI HINXTON UK; 3 September 1999 (1999-09-03), SAKAMOTO ET AL.: "Sphingomonas aromaticivorans for 16 S rRNA, complete sequence" XP002331742 Database accession no. AB0250012

## Description

### Technical Field

The present invention relates to decomposition and assimilation of polyvinyl alcohol (hereafter abbreviated as "PVA").

### Background Art

PVA is widely used for industrial applications as a water-soluble synthetic polymer. It is extensively employed as, for example, a starting material of a synthetic fiber or film, a fiber-processing agent, a paper-processing agent, an adhesive, a binder for inorganic substances, or a polymerization stabilizer for vinyl chloride resins.

Recently, concerns over environmental issues have been enhanced on a global scale, and biodegradable polymers that are decomposed by microorganisms or enzymes in nature have been actively studied. Also, a technique of decomposing various types of compounds before they are released in the natural environment to thereby decrease adverse impacts on environment has drawn attention. PVA waste liquid has been heretofore considered to be a cause of elevated COD levels in wastewater, treatment of PVA waste liquid with microorganisms such as biological sludge has been examined, and decomposing bacteria have been searched for. Examples of microorganisms that are known to be capable of decomposing PVA include *Pseudomonas* bacteria, *Enterobacter* bacteria, *Acinetobacter* bacteria, *Corynebacterium* bacteria, *Rhodococcus* bacteria, *Caseobacter* bacteria, and *Sphingomonas* bacteria (for example, JP Patent Publication (Kokoku) Nos. 55-1791 B (1980), 57-27713 B (1982), and 57-27714 B (1982) and JP Patent Publication (Kokai) Nos. 8-140667 A (1996), 7-108297 A (1995), and 2003-250527 A). Microorganisms that have been heretofore reported are bacteria that are independently capable of decomposing PVA and bacteria that are in need of symbiotic bacteria.

Kawai F: "Sphingomonads involved in the biodegradation of xenobiotic polymers" Journal of Industrial Microbiology and Biotechnology, vol. 23, no. 4-5, October 1999 (1999-10), pages 400-407 discloses PVA-utilizing Spingomonas sp. 113P3 involved in the microbial degradation of polyvinyl alcohol, which requires PQQ as essential growth factor.

Tokiwa Yutaka et al: "A modified method for isolating poly(vinyl alcohol)-degrading bacteria and study of their degradation patterns" Biotechnology Letters, vol. 23, no. 23, December 2001 (2001-12), pages 1937-1941 discloses a modified method for isolating poly(vinyl alcohol)-degrading bacteria. The degradation is carried out in the presence of PQQ.

Daugulis Andrew J et al: "Microbial degradation of high and low molecular weight polyaromatic hydrocarbons in a two-phase partitioning bioreactor by two strains of Sphingomonas sp." Biotechnology Letters, vol. 25, no. 17, September 2003 (2003-09), pages 1441-1444 describes a Sphingomonas aromaticivorans strain B0695 which is used to degrade polycyclic aromatic hydrocarbons.

Janikowski T B et al: "Use of a two-phase partitioning bioreactor for degrading polycyclic aromatic hydrocarbons by a Sphingomonas sp" Applied Microbiology and Biotechnology, vol. 59, no. 2-3, July 2002 (2002-07), pages 368-376 also describes the Sphingomonas aromaticivorans strain B0695 referred to above.

### Disclosure of the Invention

The object of the present invention is to provide PVA-decomposing bacteria that are independently highly capable of decomposing and assimilating various types of PVA derivatives, a method for decomposing and assimilating PVA with the utilization of such bacteria, and to provide a method for decomposing and assimilating PVA wherein microorganisms containing such bacteria exhibit a high level of PVA decomposing activity even in the presence of other nutrients, such as gelatin and starch, that are easily assimilable by microorganisms.

The present inventors have extensively searched for microorganisms in nature, such as in rivers, soil, and biological sludge. As a result, they have found that *Sphingomonas aromaticivorans* separated from biological sludge sampled from the Fuji Photo Film Fujinomiya Factory and microorganisms containing such bacteria are highly capable of decomposing and assimilating PVA and derivatives thereof, and use thereof enables efficient cleanup treatment of various types of PVA-containing wastes and the like. They have also found that even when nutrients such as gelatin and starch, which are easily assimilable by microorganisms, are present in wastes, the wastes can be effectively cleaned up while decomposition of PVA remains undelayed.

Thus, the present application provides a method for decomposing polyvinyl alcohol or a polyvinyl alcohol derivative, which comprises bringing bacteria that belong to the species *Sphingomonas aromaticivorans,* or a culture solution of microorganisms containing the same, into contact with polyvinyl alcohol or a polyvinyl alcohol derivative.

According to another aspect, the present application provides a method for cleaning up wastewater, waste liquid or soil, which comprises bringing bacteria that belong to the species *Sphingomonas aromaticivorans,* or a culture solution of microorganisms containing the same, into contact with wastewater, waste liquid or soil containing polyvinyl alcohol or a polyvinyl alcohol derivative.

Preferably, the bacteria that belong to the species *Sphingomonas aromaticivorans* are *Sphingomonas aromaticivorans* UF-7 (accession number: FERM BP-10187).

According to further another aspect, the present invention provides polyvinyl alcohol-decomposing bacteria which are deposited as *Sphingomonas aromaticivorans* UF-7 (accession number: FERM BP-10187).

According to further another aspect, the present invention provides a method for decomposing polyvinyl alcohol or a polyvinyl alcohol derivative, which comprises immobilizing microorganisms containing *Sphingomonas aromaticivorans* UF-7 (accession number: FERM BP-10187) on a carrier and bringing it into contact with polyvinyl alcohol or a polyvinyl alcohol derivative.

According to further another aspect, the present invention provides a method for cleaning up wastewater, waste liquid or soil, which comprises immobilizing microorganisms containing *Sphingomonas aromaticivorans* UF-7 (accession number: FERM BP-10187) on a carrier and bringing it into contact with wastewater, waste liquid or soil containing polyvinyl alcohol or a polyvinyl alcohol derivative.

Preferably, the bacteria-immobilized carrier is comprised of active carbons or crosslinked polymers.

### Brief Description of the Drawings

Fig. 1 shows the results of assaying PVA decomposition and assimilation with the utilization of *Sphingomonas aromaticivorans* UF-7 strain.
Fig. 2 shows the results of assaying PVA decomposition and assimilation with the utilization of microorganisms containing *Sphingomonas aromaticivorans* UF-7 strain.
Fig. 3 shows the results of assaying PVA decomposition and assimilation with the utilization of a carrier having microorganisms containing *Sphingomonas aromaticivorans* UF-7 strain immobilized thereon.
Fig. 4 shows the results of assaying PVA decomposition with the utilization of sampled sludge immobilized on an active carbon carrier.
Fig. 5 shows the proliferation and PVA decomposing ability of *Sphingomonas aromaticivorans* UF-7 strain in the PVA medium added with amino acids.
Fig. 6 shows the PVA decomposing ability of *Sphingomonas aromaticivorans* UF-7 strain in the PVA medium added with amino acids or methionine.

### Best Modes for Carrying out the Invention

Examples of PVA that is used in the present invention include PVA obtained by saponification of polyvinyl acetate, as well as functional PVA derivatives such as anionic, cationic, silane-reactive, or terminal-hydrophobic PVA. PVA can be decomposed and assimilated by using the bacteria of the present invention regardless of the degree of PVA polymerization or the degree of saponification, i.e., whether complete or partial saponification is involved. Examples of highly degradable PVA include those having a degree of polymerization of 300 to 3,500 and a degree of saponification of 86% to 99%. Examples of PVA derivatives include anionic PVA and terminal hydrophobic PVA.

The bacteria that are capable of decomposing and assimilating polyvinyl alcohol according to the present invention belong to the species *Sphingomonas aromaticivorans*.

The species *Sphingomonas aromaticivorans*, which is capable of decomposing and assimilating polyvinyl alcohol, has been extensively searched for in nature. For example, it can be separated in the following manner.

The species *Sphingomonas aromaticivorans* which is capable of decomposing and assimilating polyvinyl alcohol according to the present invention, is separated, for example, in the following manner. Specifically, samples such as those from rivers, biological sludge, soil, marine water or lake water are filtered through a membrane filter (pore diameter: 0.1 µm to 0.5 µm), and the membrane filter is soaked in a PVA liquid medium and subjected to shaking culture to evaluate the presence of PVA-decomposing bacteria. Thereafter, the culture solution is dispersed on an adequate medium to form colonies. The resulting colonies are inoculated on a low nutrient medium containing PVA, and bacteria capable of decomposing PVA may be selected and then separated.

An example of the bacteria capable of decomposing and assimilating PVA and derivatives thereof according to the present invention is *Sphingomonas aromaticivorans* UF-7 strain. This *Sphingomonas aromaticivorans* UF-7 strain is deposited at the International Patent Organism Depositary of the National Institute of Advanced Industrial Science and Technology (Tsukuba Central 6, 1-1-1 Higashi, Tsukuba, Ibaraki, Japan) as of February 4, 2004, under the accession number FERM P-19664, and was transferred to International deposition under Budapest treaty as of December 21, 2004 under the accession number FERM BP-10187. Mycological properties and the results of genetic analysis of the *Sphingomonas aromaticivorans* UF-7 are described below. (Mycological properties)

Properties of medium: LB agar medium (Sambrook, J., E. F. Fritsch, and T. Maniatis, 1989, Molecular cloning: a laboratory manual, 2nd ed., Cold Spring Harbor Laboratory Press, N.Y.); yellow, slightly convex, and round colonies are formed.

### (Genetic analysis)

### Analysis of 16 SrDNA

The UF-7 strain was inoculated on LB agar medium, DNA was extracted from a culture product at 30°C, the extracted DNA was amplified by PCR, and the PCR product was purified. Thereafter, cycle sequencing was carried out using the BigDye^{™} Terminator v. 3.1 (Applied Biosystems) to determine the nucleotide sequence of 16 SrDNA, and 16 SrDNA was analyzed using the FASTA Program of the DNA Data Bank of Japan (http://www.ddbj.nig.ac.jp/) on the web. As a result, the 16 SrDNA of the UF-7 strain of the present invention was found to have 98.2% homology to that of *Sphingomonas aromaticivorans* (M. Sakamoto et al., DDBJ/EMBL/GenBank databases direct submission; Accession No. AB025012).

In principle, the aforementioned bacteria are cultured in accordance with, for example, a culture technique for microorganisms that belong to the genus *Sphingomonas*. In general, bacteria are preferably cultured under aerobic conditions, such as with shaking culture via liquid culture or aeration agitation culture.

A variety of synthetic media, semi-synthetic media, naturally-occurring media, and the like for aerobic bacteria can be employed for culture. Examples thereof include meat extract and peptone. Also, the aforementioned bacteria are preferably cultured in the presence of methionine.

The pH level of a medium is preferably between 6 and 8. The culture temperature is preferably kept at a temperature where *Sphingomonas aromaticivarans* is grown well. This is generally about 15°C to 42°C, and particularly preferably about 25°C to 38°C. A culture period is generally for 2 days to 1 week under aerobic conditions regardless of the type of culture conducted, i.e., whether liquid shaking culture or aeration agitation culture is employed. It should be noted that such culture conditions can be adequately altered depending on the types and properties of microorganisms to be used, external conditions, and other conditions.

The bacteria according to the present invention can effectively decompose and assimilate PVA and PVA derivatives as described in the Examples below. Accordingly, bacteria that belong to the species *Sphingomonas aromaticivorans*, microorganisms containing the same, or bacterial components thereof can be brought into contact with industrial wastes, such as household effluents, industrial effluents, or waste liquids, or PVA-containing substances such as soil, to clean up them. The bacterial components include cultured products of bacteria that belong to the genus *Sphingomonas*, immobilized bacteria, bacterial extracts, immobilized bacterial extracts, bacterial liquid extracts, culture solutions, and culture filtrates. Examples of carriers that can be used for immobilizing bacteria include active carbons and crosslinked polymers.

Examples of methods for decomposing PVA with the utilization of the microorganisms according to the present invention in the case of wastewater cleanup include a means for installing a processor having the bacteria of the present invention, i.e., *Sphingomonas aromaticivorans*, or microorganisms containing the same immobilized thereon, at individual sources, and a means for adding the bacteria of the present invention or microorganisms containing the same to an activated sludge tank or the like in a wastewater plant. An example of such method in the case of soil cleanup is a means for applying the bacteria of the present invention or microorganisms containing the same to soil. In the case of environmental cleanup, a means for applying the bacteria of the present invention or microorganisms containing the same to river or lake in nature can be employed. Such PVA decomposition treatment is carried out at a pH 4 to 10 and preferably pH 5 to 8, and at a processing temperature of 5°C to 40°C and preferably of 15°C to 35°C, for 0.5 hour to 14 days in general and preferably for 1 hour to 7 days.

The present invention is hereafter described in greater detail with reference to the following examples.

### Examples

### Example 1: Isolation of Sphingomonas aromaticivorans UF-7 strain

100 ml of PVA medium (Table 1) was prepared, wild-type *E. coli* MG1655 strain having no PVA decomposition ability was inoculated in order to remove impurities contained in PVA used as a starting material, such as acetate, and shaking culture was carried out at 30°C overnight. The culture solution was filtered through a membrane filter (pore diameter: 0.2 µm) for sterilization. A suspension of PVA waste sludge (50 µl) was inoculated on the filtered PVA culture medium, and shaking culture was carried out at 30°C for 10 days. LB agar medium was coated with a part of the culture solution, the resultant was cultured at 30°C for 1 week, single colonies were selected from a plate on which colony growth had been observed, and the ability for PVA decomposition was confirmed and the identification test were carried out. Thus, *Sphingomonas aromaticivorans* UF-7 strain was obtained.

**Table 1: Components of PVA medium**

| Components | Per liter of medium |
|---|---|
| PVA | 1 g |
| Ammonium sulfate | 1 g |
| Magnesium sulfate | 1 g |
| Sodium chloride | 0.1 g |
| Iron (II) sulfate heptahydrate | 0.01 g |
| Dried yeast extract | 0.05 g |
| Monobasic potassium phosphate | 0.4 g |
| Dibasic potassium phosphate | 3.2 g |

### Example 2: Decomposition and assimilation of PVA utilizing Sphingomonas aromaticivorans UF-7 strain

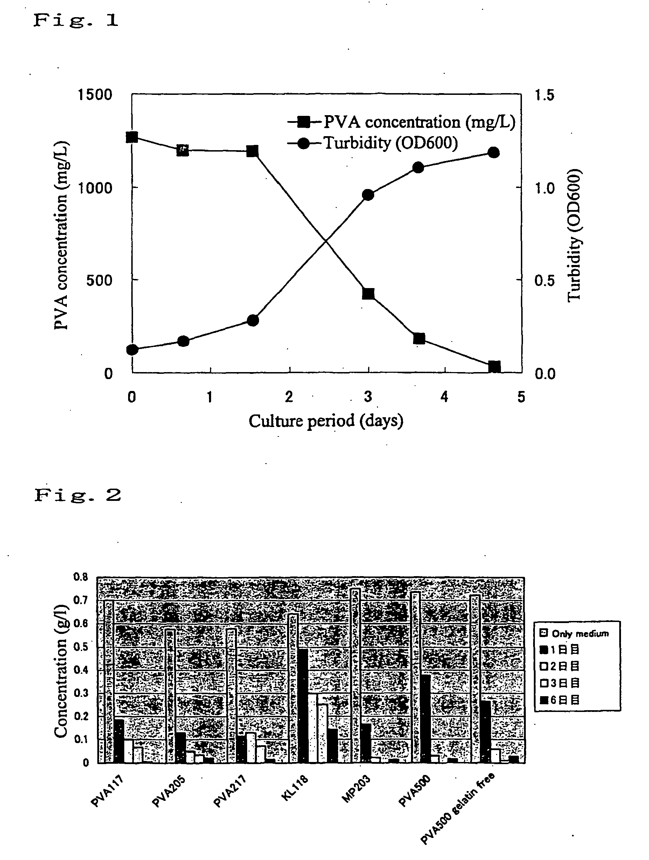

In preculture, UF-7 strains grown in an LB agar medium were inoculated on 4 ml of LB liquid medium using an inoculating loop, and shaking culture was carried out at 30°C for approximately 4 days. Bacteria were separated from the culture solution, washed several times with modified PVA medium (Table 2), inoculated on a fresh modified PVA medium, and subjected to shaking culture at 30°C, Part of the culture solution was removed with the elapse of time, and turbidity (OD 600) and PVA concentration thereof were measured (Kim, B. C., C. K. Sohn, S. K. Lim, J. W. Lee, and W. Park, 2003, Degradation of polyvinyl alcohol by Sphingomonas sp. SA3 and its symbiote, J. Ind. Microbiol. Biotechnol., 30, 70-74). The results are shown in Fig. 1. As is apparent from the results shown in Fig. 1, the UF-7 strain was capable of effectively decomposing PVA by itself without the necessity of PQQ addition.

**Table 2: Components of modified PVA medium**

| Components | Per liter of medium |
|---|---|
| PVA | 1 g |
| Ammonium sulfate | 1 g |
| Magnesium sulfate | 1 g |
| Sodium chloride | 0.1 g |
| Monobasic potassium phosphate | 0.4 g |
| Dibasic potassium phosphate | 3.2 g |
| Calcium chloride | 60 µg |
| Cobalt (II) chloride hexahydrate | 7 µg |
| Manganese (II) chloride tetrahydrate | 16 µg |
| Ammonium molybdate tetrahydrate | 4 µg |
| Boric acid | 25 µg |
| Copper (II) sulfate pentahydrate | 2.5 µg |
| Zinc sulfate heptahydrate | 3 µg |
| Iron (II) sulfate heptahydrate | 3 mg |
| Mixed solution containing 20 types of standard amino acids (1 g/l each) | 10 ml |

### Example 3: Decomposition and assimilation of PVA utilizing microorganisms containing Sphingomonas aromaticivorans UF-7

A culture solution (1 ml) of microorganisms containing *Sphingomonas aromaticivorans* UF-7 sampled from biological sludge was added to 20 ml of medium containing various types of PVAs (Table 3), shaking culture was carried out at 28°C at 140 rpm, part of the culture solution was removed with the elapse of time as with the case in Example 2, and the PVA concentration thereof was measured. The results are shown in Fig. 2.

**Table 3: Medium for studying decomposition of various types of PVA**

| Compound | Weight (g) |
|---|---|
| PVA* | 0.5 |
| Bacto Gelatin | 0.5 |
| (NH₄)₂SO₄ | 1 |
| KH₂PO₄ | 0.4 |
| K₂HPO₄ | 3.2 |
| MgSO₄ | 0.98 |
| NaCl | 0.1 |
| FeSO₄ · 7H₂O | 0.01 |
| Yeast extract | 1 |

| | |
|---|---|
| *PVA (PVA500; Wako Pure Chemical Industries, Ltd.; others: Kuraray Co., Ltd.) (i) PVA-117 (saponification degree of 98 to 99 mol %; polymerization degree of 1700) (ii) PVA-205 (saponification degree of 87 to 89 mol %; polymerization degree of 500) (iii) PVA-217 (saponification degree of 87 to 89 mol %; polymerization degree of 1700) (iv) K-118 (saponification degree of 98 to 99 mol %; polymerization degree of 1800) (v) MP-203 (saponification degree of 87 to 89 mol %; polymerization degree of 300) (vi) PVA500 (Wako; saponification degree of >96 mol %; polymerization degree of 500) (vii) PVA500 (Wako) without Bacto Gelatin KL- 118: anionic PVA; MP-203: terminal hydrophobic PVA | |

As is apparent from the results shown in Fig. 2, the microorganisms of the present invention exhibited a high level of PVA decomposition activity regardless of the types of PVA involved. Decomposition of PVA was not delayed even in the presence of gelatin, which is easily assimilable by microorganisms.

### Example 4: Decomposition and assimilation of PVA utilizing a carrier having microorganisms containing Sphingomonas aromaticivorans UF-7 immobilized thereon

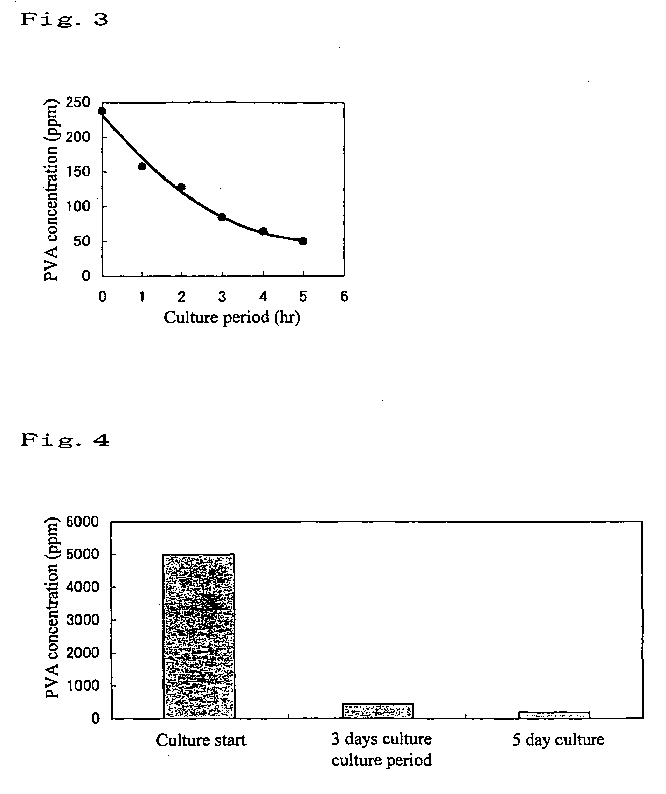

A culture solution of microorganisms containing *Sphingomonas aromaticivorans* UF-7 sampled from biological sludge was immobilized on 35 g of carrier (Kuragel, Kuraray Co., Ltd.), 100 ml of the medium shown in Table 4 was added, and shaking culture was carried out at 28°C at 140 rpm. Part of the culture solution was removed with the elapse of time as with the case in Example 2, and the PVA concentration thereof was measured. The results are shown in Fig. 3. As is apparent from the results shown in Fig. 3, it was confirmed that PVA could be processed within a short period of time.

**Table 4: Medium for studying decomposition of various types of PVAs**

| Compound | Weight (g) |
|---|---|
| PVA500 | 0.25 |
| (NH₄)₂SO₄ | 1 |
| KH₂PO₄ | 0.4 |
| K₂HPO₄ | 3.2 |
| MgSO₄ | 0.98 |
| NaCl | 0.1 |
| FeSO₄ · 7H₂O | 0.01 |
| Yeast extract | 1 |

### Example 5: Decomposition treatment of PVA utilizing sampled sludge immobilized on active carbon carrier

Microorganisms containing *Sphingomonas aromaticivorans* UF-7 strain were cultured in a PVA-containing medium for approximately 2 weeks together with an active carbon carrier for water treatment (BCP, Denka Engineering), and bacteria were immobilized. The following experiment was carried out using this carrier.

A medium (20 ml) containing 0.5% of PVA and 0.1% of yeast extract was added to 6 ml of the microorganism-immobilized BCP, and shaking culture was carried out at 28°C at 140 rpm. The culture solution was sampled, and the PVA concentration in the centrifuged supernatant was quantified by the Finley method (KI/I₂-H₃BO₃, 690 nm). The results are shown in Fig. 4. The PVA concentration was lowered to a concentration that was 1/10 or less that of the initial level in 3 days. This indicates that the effects of highly densified bacteria are exhibited by immobilization thereof on a carrier.

### Example 6: Study as to amino acid requirement

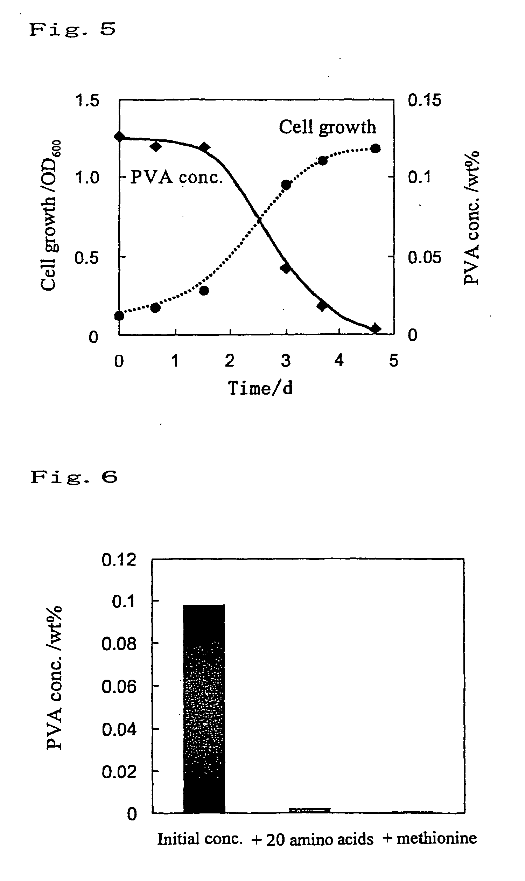

UF-7 strain was cultured alone in the modified PVA medium added with 20 types of standard amino acids (each amino acid 0.01%, total 0.20%). As a result, the proliferation of the strain was observed, and it was confirmed that about 0.12 % PVA was almost completely decomposed in 5 days (Fig.5). Thus, it was found that a component required for the UF-7 strain is present in 20 types of the standard amino acids.

The role of the standard amino acids was examined. Two glucose media containing no dried yeast extract were prepared, and one of them was added with an appropriate amount of 20 types of the standard amino acids. The UF-7 strains were cultured alone for 1 week in these two types of the media, and the proliferation of the strains was observed. As a result, the proliferation of the strain was observed in the medium added with amino acids, but no proliferation was observed in the medium added with no amino acid. These results indicate that 20 types of the standard amino acids are necessary for the PVA decomposition, as well as for the proliferation of UF-7 strain as nutrients.

Next, it was examined which amino acid among the 20 types of the standard amino acids is necessary for the UF-7 strain. Two modified PVA media were prepared. One of them was added with an appropriate amount of 10 types of the standard amino acids, and the other of them was added with an appropriate amount of the other remaining 10 types of the standard amino acids. The UF-7 strains were cultured alone for 1 week in these two types of the media, and the strain weight and the PVA concentration change were measured. The amino acid group where the strain proliferation and PVA decomposition activity were observed, was selected, and the similar operation was repeated as in the above, so as to identify an essential amino acid. As a result, it was confirmed that the strain grew and PVA was decomposed by adding only methionine (Fig.6). The above results indicate that methionine is necessary for the UF-7 strain. As a result, the proliferation of the strain was observed in the medium added with amino acids, but no proliferation was observed in the medium added with no amino acid. These results indicate that 20 types of the standard amino acids are necessary for the PVA decomposition, as well as for the proliferation of UF-7 strain as nutrients.

**Modified PVA medium**

| | |
|---|---|
| PVA | 1.0g |
| Solution I (see below) | 50ml |
| Solution II (see below) | 50ml |
| Trace metal (see below) | 2ml |
| H₂O | to 1.0 L |

**Solution I**

| | |
|---|---|
| (NH₄)₂SO₄ | 20 g/l |
| MgSO₄ | 19.6 g/l |
| NaCl | 2.0 g/l |

**Solution II**

| | |
|---|---|
| KH₂PO₄ | 8.0g/l |
| K₂HPO₄ | 64 g/l |

**Trace metal**

| | |
|---|---|
| CaCl₂ | 250 µM |
| (NH₄)₆Mo₇O₂₄ | 1.5 µM |
| H₃BO₃ | 200 µM |
| CoCl₂ | 15 µM |
| CuSO₄ | 5 µM |
| MnCl₂ | 40 µM |
| ZnSO₄ | 5 µM |
| FeSO₄ | 5 mM |

**Additives to modified PVA medium**

| Additives | Stock Conc. | Final Conc. | Amount (4ml/100ml) |
|---|---|---|---|
| peptone | 5% (50 times) | 0.1% | 80µl /2 ml |
| Amino acids (20 types) | 10% (50 times) | 0.2% | 80 µl /2 ml |
| Amino acids (various types) | 0.5% (50 times) | 0.01% | 90 µl / 2 ml |
| Amino acids (poorly soluble) | 0.2% (20 times) | 0.01% | 200 µl / 5 ml |

### Effect of the Invention

The method for decomposing PVA according to the present invention is effective for decomposition and assimilation of PVA in the clean up and composting treatment of various forms of wastewater, waste liquid soil, and the like.

## Claims

1. A method for decomposing polyvinyl alcohol or a polyvinyl alcohol derivative, which comprises bringing bacteria that belong to the Sphingomonas aromaticivorans strain UF-7 (accession no. FERM BP-10187), or a culture solution of microorganisms containing the same, into contact with polyvinyl alcohol or a polyvinyl alcohol.

2. A method for cleaning up wastewater, waste liquid or soil, which comprises bringing bacteria as defined in claim 1, or a culture solution of microorganisms containing the same, into contact with wastewater, waste liquid or soil containing polyvinyl alcohol or a polyvinyl alcohol derivative.

3. Polyvinyl alcohol-decomposing bacteria which are deposited as *Sphingomonas aromaticivorans* UF-7 (accession no. FERM BP-10187).

4. A method for decomposing polyvinyl alcohol or a polyvinyl alcohol derivative, which comprises immobilising microorganisms containing Sphingomonas aromaticivorans UF-7 (accession no. FERM BP-10187) on a carrier and bringing it into contact with polyvinyl alcohol or a polyvinyl alcohol derivative.

5. A method for cleaning up wastewater, waste liquid or soil, which comprises immobilising microorganisms containing *Sphingomonas aromaticivorans* UF-7 (accession no. FERM BP-10187) on a carrier and bringing it into contact with wastewater, waste liquid or soil containing polyvinyl alcohol or a polyvinyl alcohol derivative.

6. The method according to claim 4 or 5, wherein the bacteria-immobilised carrier is comprised of active carbons or crosslinked polymers.

## Patentansprüche

1. Verfahren zum Zersetzen von Polyvinylalkohol oder einem Polyvinylalkoholderivat, welches das Inkontaktbringen von Bakterien, die zu dem Sphingomonas aromaticivorans-Stamm UF-7 (Zugangsnummer FERM BP-10187) gehören, oder einer Kulturlösung von Mikroorganismen, welche diese enthalten, mit Polyvinylalkohol oder einem Polyvinylalkoholderivat umfasst.

2. Verfahren zum Säubern von Abwasser, flüssigem Müll oder Boden, welches das Inkontaktbringen von Bakterien, welche in Anspruch 1 definiert sind, oder einer Kulturflüssigkeit von Mikroorganismen, welche diese enthalten, mit Abwasser, flüssigem Müll oder Boden umfasst, welche Polyvinylalkohol oder ein Polyvinylalkoholderivat enthalten.

3. Polyvinylalkohol-zersetzendes Bakterium, welches hinterlegt ist als Sphingomonas aromaticivorans UF-7 (Zugangsnummer FERM BP-10187).

4. Verfahren zum Zersetzen von Polyvinylalkohol oder einem Polyvinylalkoholderivat, welches das Immobilisieren von Mikroorganismen auf einem Träger umfasst, welche Sphingomonas aromaticivorans UF-7 (Zugangsnummer FERM BP-10187) enthalten und Inkontaktbringen mit Polyvinylalkohol oder einem Polyvinylalkoholderivat.

5. Verfahren zum Reinigen von Abwasser, flüssigem Müll oder Boden, welches das Immobilisieren von Mikroorganismen, welche Sphingomonas aromaticivorans UF-7 enthalten, (Zugangsnummer FERM BP-10187) auf einem Träger umfasst und Inkontaktbringen davon mit Abwasser, flüssigem Müll oder Boden, welche Polyvinylalkohol oder ein Polyvinylalkoholderivat enthalten.

6. Verfahren gemäß Anspruch 4 oder 5, wobei der bakterienimmobilisierte Träger aus Aktivkohle oder vernetzten Polymeren besteht.

## Revendications

1. Procédé pour décomposer l'alcool de polyvinyle ou un dérivé de l'alcool de polyvinyle, qui comprend l'étape consistant à amener des bactéries qui appartiennent à la souche Sphingomonas aromaticivorans UF-7 (n° d'accession FERM BP-10187) ou une solution de culture de microorganismes les contenant, en contact avec l'alcool de polyvinyle ou un alcool de polyvinyle.

2. Procédé pour nettoyer de l'eau usée, un rejet liquide ou un sol, qui comprend l'étape consistant à amener des bactéries telles que définies à la revendication 1, ou une solution de culture de microorganismes les contenant, en contact avec l'eau usée, le rejet liquide ou le sol contenant l'alcool de polyvinyle ou un dérivé de l'alcool de polyvinyle.

3. Bactéries décomposant l'alcool de polyvinyle, qui sont déposées sous le nom de Sphingomonas aromaticivorans UF-7 (n° d'accession FERM BP-10187).

4. Procédé de décomposition de l'alcool de polyvinyle ou d'un dérivé de l'alcool de polyvinyle, qui comprend les étapes consistant à immobiliser des microorganismes contenant Sphingomonas aromaticivorans UF-7 (n° d'accession FERM BP-10187) sur un support, et à les amener en contact avec l'alcool de polyvinyle ou un dérivé de l'alcool de polyvinyle.

5. Procédé pour purifier de l'eau usée, un rejet liquide ou un sol, qui comprend les étapes consistant à immobiliser des microorganismes contenant Sphingomonas aromaticivorans UF-7 (n° d'accession FERM BP-10187) sur un support, et à les amener en contact avec l'eau usée, le rejet liquide ou le sol contenant de l'alcool de polyvinyle ou un dérivé de l'alcool de polyvinyle.

6. Procédé selon la revendication 4 ou 5, dans lequel le support d'immobilisation des bactéries se compose de charbons actifs ou de polymères réticulés.
